# EUROPÄISCHE PATENTSCHRIFT

(11) **EP 1 044 692 B1**
(45) Veröffentlichungstag und Bekanntmachung des Hinweises auf die Patenterteilung: **19.02.2003**
(21) Anmeldenummer: 00107004.4
(22) Anmeldetag: 30.03.2000
(51) Int. Cl.: A61K 51/12

(54) **Verfahren und Vorrichtung zum trockenen Anbringen von Substanzen an inhalierbare pulverförmige Trägerstoffe**
Process and device for dry application of products to inhalable powdery supports
Préparation et dispositif pour application sèche de produits à des supports pulvérulents

(30) Priorität: 16.04.1999 DE 19917347
(43) Veröffentlichungstag der Anmeldung: 18.10.2000
(73) Patentinhaber: GSF-Forschungszentrum für Umwelt und Gesundheit GmbH, 85764 Oberschleissheim (DE)
(72) Erfinder: Scheuch, Gerhard, 35285 Gemünden (DE); Sommerer, Knut, 81241 München (DE)
(74) Vertreter: Reinhard - Skuhra - Weise & Partner

(56) Entgegenhaltungen:
- DE-A- 3 122 724
- US-A- 4 057 616
- US-A- 5 829 436
- DATABASE CHEMABS [Online] CHEMICAL ABSTRACTS SERVICE, COLUMBUS, OHIO, US; FEW, J. D. ET AL: "Preparation of technetium-99m labeled particles for aerosol studies" retrieved from STN Database accession no. 74:83819 XP002143425 & RADIOCHEM. RADIOANAL. LETT. (1970), 5(6), 275-7 ,

## Beschreibung

Die Erfindung betrifft ein Verfahren und eine Vorrichtung zum trockenen Anbringen von Substanzen, insbesondere von Markierungssubstanzen und/oder aktiv wirksamen Substanzen, an inhalierbare pulverförmige Trägerstoffe.

Inhalierbare Trockenpulver werden zum Teil aus Lactose, Liposomen oder anderen Trägersubstanzen hergestellt. Für die Überprüfung, wieviele inhalierte Partikel in der Lunge abgelagert werden, ist es beispielsweise aus der US 5,829,436 bekannt, an diese pulverförmigen Trägerstoffe bzw. Aerosolteilchen eine Markierung anzubringen, um die Lage und Verteilung der Partikel von außen nachweisen zu können. Beispielsweise aus der US 4,057,616 ist es bekannt, als Markierung üblicherweise radioaktive Substanzen, wie beispielsweise Natrum-Pertechnetat (99mTc) Tc04 zu verwenden. Statt dieser radiaktiven Markierungssubstanzen ist es jedoch auch möglich, künftig magnetische Marker oder Kontrastmittel einzusetzen, die mit kernmagnetischer Resonanzspektrographie (NMR) nachweisbar sind. Statt dieser Markierung von pulverförmigen Trägerstoffen zum externen qualitativen und quantitativen Nachweis von abgelagerten Partikel in der Lunge können auch kleinste Mengen wirksame Substanzen, insbesondere Medikamente an pulverförmige inhalierbare Trägerstoffe angebracht werden. Des weiteren kann auch das Anbringen derartiger wirksamer Substanzen in kleinen Mengen mit einer Markierung kombiniert sein. Beispielsweise aus der DE 31 22 724 ist es bekannt, kleine Partikel eines pulverförmigen Trägerstoffs in eine Lösung zu geben, in der sich die Markierungssubstanz befindet und dann eintrocknet. Dabei besteht jedoch nachteiligerweise das Problem, dass insbesondere bei wasserlöslichen Substanzen bzw. Trägerstoffen die Eigenschaften der Trägerstoffpartikel verlorengehen oder sich diese Partikel vollständig auflösen.

Der Erfindung liegt die Aufgabe zugrunde, ein Verfahren und eine Vorrichtung der eingangs genannten Gattung verfügbar zu machen, bei dem die Beibehaltung der inhalierbaren pulverförmigen Trägerstoffe und deren Eigenschaften bei dem Anbringen von Markierungssubstanzen und/oder aktiv wirksamen Substanzen erreicht wird.

Erfindungsgemäß wird diese Aufgabe verfahrensseitig durch die im Patentanspruch 1 genannten Merkmale gelöst. Bevorzugte Merkmale, die dieses Verfahren vorteilhaft weiterbilden, sind in den nachgeordneten Verfahrensansprüchen 2 bis 5 angegeben.

Vorrichtungsseitig wird diese Aufgabe durch die Merkmale des Patentanspruchs 6 gelöst. Bevorzugte weitere Ausgestaltungen der Erfindung sind in den nachgeordneten Vorrichtungsansprüchen enthalten.

Nach dem erfindungsgemäßen Verfahren wird somit das trockene Anbringen von Substanzen an inhalierbare pulverförmige Trägerstoffe vorgenommen, indem zunächst die anzubringende Substanz einer Flüssigkeit gelöst, anschließend das Fluid vernebelt und die entstandenen Aerosoltröpfchen vollständig zur Erzeugung von Aerosolteilchen getrocknet werden. Danach wird ein verwirbelndes Kontaktieren der Aerosolteilchen mit dem pulverförmigen Trägerstoff vorgenommen, wobei vorteilhaft die feinen Aerosolteilchen an den zu markierenden Pulverteilchen haften.

Gemäß einer bevorzugten Ausgestaltung der Erfindung wird vorteilhaft zur Vermeidung einer Beeinflussung der zu markierenden Pulverteilchen des pulverförmigen Trägerstoffs die Feuchte der Aerosolteilchen vor dem verwirbelnden Kontaktieren kontrolliert, wobei bevorzugt die Feuchte der Aerosolteilchen auf den jeweiligen Trägerstoff eingestellt wird.

Nach einer bevorzugten Ausgestaltung des erfindungsgemäßen Verfahrens wird das verwirbelnde Kontaktieren durch Ansaugen von zirkulär geführten Aerosolteilchen durch den Trägerstoff vorgenommen, wobei vorzugsweise das Ansaugen der Aerosolteilchen über einen Rückhaltefilter durchgeführt wird. Hierdurch wird vorteilhaft der pulverförmige Trägerstoff aufgewirbelt, und es kommt durch die sich zirkulär, ähnlich wie bei einem Zyklonabscheider, geführten Aerosolteilchen zu einer besonders innigen Vermischen und dem gewünschten trockenen Anbringen der Markierungssubstanzen und/oder aktiv wirksamen Substanzen.

Gemäß der erfindungsgemäßen Vorrichtung sind apparativ ein Aerosolgenerator und eine Mischkammer vorgesehen, wobei der Aerosolgenerator einen Düsenvernebler für die in einer Flüssigkeit gelöste anzubringende Substanz und eine Trocknungseinrichtung für die erzeugten Aerosolteilchen aufweist. Die Mischkammer dient zur Aufnahme von pulverförmigem Trägerstoff und zu dessen verwirbelnden Kontaktierung mit zirkulär eingeleiteten trockenen Aerosolteilchen.

Die Substanz, beispielsweise Natrium-Pertechnetat, mit der die pulverförmigen Trägerstoffe markiert werden sollen, ist dabei in einer Flüssigkeit, beispielsweise Wasser oder Alkohol, gelöst, wobei in dem Aerosolgenerator mit einem herkömmlichen Düsen- oder Ultraschallvernebler eine Vernebelung erfolgt. Die Vernebelung kann mit einem jeden System erfolgen, das eine Aerosolerzeugung aus einer Flüssigkeit ermöglicht. Der Aerosolgenerator besitzt vorteilhaft ein großes Volumen und eine Trokkensubstanz, beispielsweise Silica-Gel, um die erzeugten Aerosoltröpfchen vollständig zu trocknen, ehe sie den Aerosolgenerator verlassen. Es lassen sich auf diese Weise sehr kleine Aerosolteilchen mit einer Teilchengröße zwischen 1 nm und 500 nm herstellen.

Diese trockenen Aerosolteilchen werden dann in die angeschlossene Mischkammer geleitet, in der sich der inhalierbare pulverförmige Trägerstoff befindet, an dem die trockenen Aerosolteilchen angebracht werden sollen.

Für das Einleiten trockener Aerosolteilchen und die Verwirbelung in der Mischkammer ist nach einer bevorzugten Ausgestaltung der Erfindung eine Vakuumpumpe vorgesehen, die über eine Filteranordnung mit der Mischkammer verbunden ist. Durch die weiterhin in günstiger Weise vorgesehene exzentrische Einleitung der trockenen Aerosolteilchen in die Mischkammer, welche vorzugsweise im unteren Bereich der Mischkammer mit schräg nach unten geneigter Orientierung vorgesehen ist, wird der inhalierbare pulverförmige Trägerstoff aufgewirbelt, und die feinen Aerosolteilchen haften an den pulverförmigen Teilchen des Trägerstoffs.

Zur Kontrolle der Feuchte der getrockneten Aerosolteilchen ist vorzugsweise ein Feuchtesensor, insbesondere ein Hygrometer, vorgesehen, wobei der Feuchtesensor vorzugsweise in einer Verbindungsleitung zwischen Aerosolgenerator und Mischkammer angeordnet ist und bevorzugt die Feuchte der getrockneten Aerosolteilchen zur Anpassung an die jeweilige Trägersubstanz mittels eines entsprechenden Ein- und Ausschaltens des Düsenverneblers im Aerosolgenerator einstellbar ist.

Gemäß einer weiteren bevorzugten Ausgestaltung der Erfindung ist die Mischkammer mittels eines Deckels mit einem Öffnungsstutzen für den Anschluß einer Vakuumpumpe abgedichtet verschließbar, wobei der Deckel teilweise in einen oberen Abschnitt der Mischkammer einsetzbar und mittels einer Überwurfmutter auf der Mischkammer befestigbar ist. Hierdurch läßt sich die Mischkammer leicht mit dem inhalierbaren pulverförmigen Trägerstoff zur Vorbereitung des trockenen Anbringens von Substanzen füllen und nach dem Anbringen problemlos entleeren.

Gemäß einer weiteren bevorzugten Ausgestaltung der Erfindung ist eine Filteranordnung zwischen Deckel und Mischkammer vorgesehen, wobei die Filteranordnung einen Aufbau aus einem Feinfilter mit einer Porengröße von ca. 1-3 µm, vorzugsweise in Form eines Cellulosefilters, und aus einem Stützfilter hoher Festigkeit und einer Größe der Öffnungen zwischen 50-500 µm, vorzugsweise in Form einer Metallfolie, aufweist. Der Feinfilter findet dabei vorteilhaft das Entweichen von Pulver aus der Mischkammer und wird durch den Stützfilter, der den Feinfilter an der der Vakuumpumpe zugewandten Seite kontaktiert, vor dem Zerreißen geschützt.

Gemäß einer weiteren Ausgestaltung der Erfindung ist der Aerosolgenerator zur Erzeugung einer Partikelgröße < 0,5 um mit einer relativen Feuchte < 50%, vorzugsweise < 30%, vorgesehen.

Nachfolgend wird die Erfindung anhand der beigefügten Zeichnungen näher erläutert. Es zeigen:
- Fig. 1: eine schematische Darstellung von verschiedenen Vorrichtungsteilen;
- Fig. 2: einen Vertikalschnitt durch eine Ausbildung der Mischkammer;
- Fig. 3: eine Explosionsschnittansicht der Mischkammer von Fig. 2;
- Fig. 4: eine Draufsicht auf den unteren Teil der Mischkammer von Fig. 2; und
- Fig. 5: eine Darstellung einer gravimetrisch und radioaktiv gemessenen Partikelverteilung.

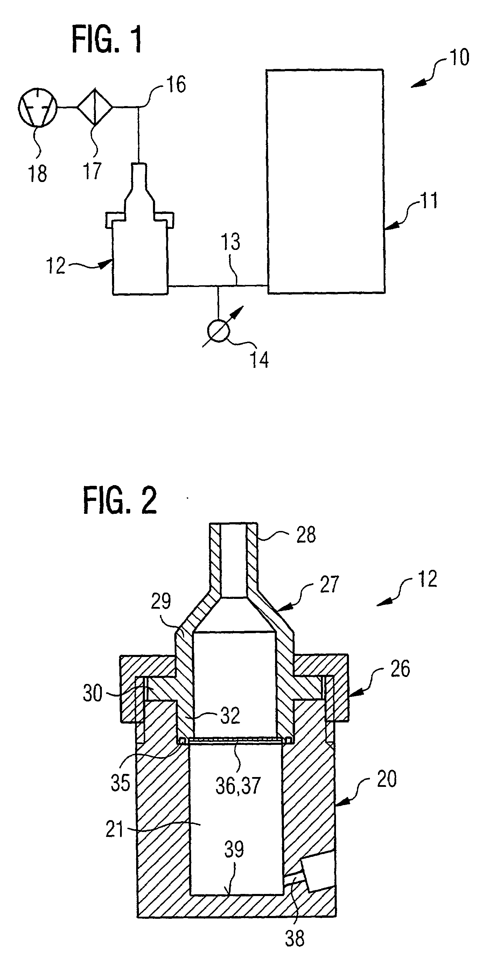

In Fig. 1 ist schematisch eine Ausgestaltung der erfindungsgemäßen Vorrichtung 10 dargestellt. Die Vorrichtung 10 besteht in Fig. 1 aus einem Aerosolgenerator 11 und einer Mischkammer 12, die an dem Aerosolgenerator 11 über eine Verbindungsleitung 13 zur Einleitung getrockneter Aerosolteilchen in die Mischkammer 12 verbunden ist. In die Leitung 13 ist ein Feuchtesensor 14 eingeschaltet, durch den die Feuchtigkeit der getrockneten Aerosolteilchen gemessen werden kann und der einen nicht dargestellten herkömmlichen Düsenvernebler in dem Aerosolgenerator 11 zur individuellen Einstellung der Feuchte in nicht dargestellter Weise ein- und ausschalten kann.

Am oberen Ende der Mischkammer ist eine Leitung 16 angeschlossen, die über eine Filteranordnung 17 zu einer Vakuumpumpe 18 führt.

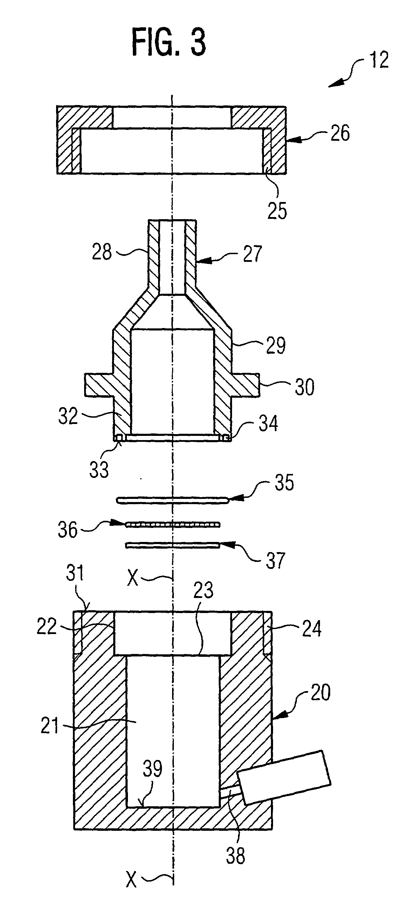

In Fig. 2 ist eine über Fig. 1 abgewandelte Mischkammer 12 mit einem vertikalen Schnitt dargestellt. Die Abwandlung gegenüber der Mischkammer in Fig. 1 besteht darin, daß die Filteranordnung 17 im Bereich des Auslasses der Mischkammer angeordnet ist, wie nachfolgend noch näher erläutert wird. Die Mischkammer 12 gemäß Fig. 2 und 3 besteht aus einem hohlzylindrischen Grundkörper 20, in dessen Inneren die eigentliche Kammer zur Aufnahme von pulverförmigem Trägerstoff und zur Verwirbelung gebildet ist. Die untere Seite des Grundkörpers 20 ist geschlossen. Der Grundkörper 20 ist, wie im Zusammenhang mit Fig. 3 ersichtlich, nach oben offen, wobei sich der zylinderförmige Innenraum 21 über einen radialen Schulterabschnitt 23 zu einem offenen, im Durchmesser größeren Einsetzabschnitt 22 erweitert. Im Bereich des Einsetzabschnitts 22 ist an dem Grundkörper 20 ein Außengewinde 24 vorgesehen, auf das ein Innengewinde 25 einer Überwurfmutter 26, wie in Fig. 2 gezeigt, geschraubt ist.

Mit 27 ist ein hohler Deckel bezeichnet, der an seiner Oberseite einen Ansaugstutzen 28 für die Vakuumpumpe 18 aufweist, welcher sich konisch zu einem Hohlzylinderabschnitt 29 erweitert, der einen radialen Anlageflansch 30 zur Auflage auf die oberseitige Fläche 31 des Grundkörpers 20 besitzt. Unterhalb des Flansches 30 ist ein Einsetzabschnitt 32 gebildet, der in eine radiale Anlagefläche 33 für die Schulter 23 mündet, wie in Fig. 2 gezeigt. In der Fläche 33 befindet sich eine Nut 34 für einen O-Ring 35 und eine im Durchmesser kleinere schulterförmige Ausnehmung zur Aufnahme einer Filteranordnung aus einem Stützfilter 36 und einem Feinfilter 37. Bei dem in Fig. 2 gezeigten Zusammenbauzustand der Mischkammer 12 liegen die Filter 36 und 37 aneinander, und das Innere 21 des Grundkörpers 20 ist über die aneinanderliegenden Filter 36 und 37 nach oben hin abgeschlossen. Aus Fig. 2 ist weiterhin erkennbar, wie die Überwurfmutter 26 auf dem Flansch 30 des Deckels 27 aufliegt und mit ihrem Innengewinde 25 auf das Außengewinde 24 des Grundkörpers geschraubt ist.

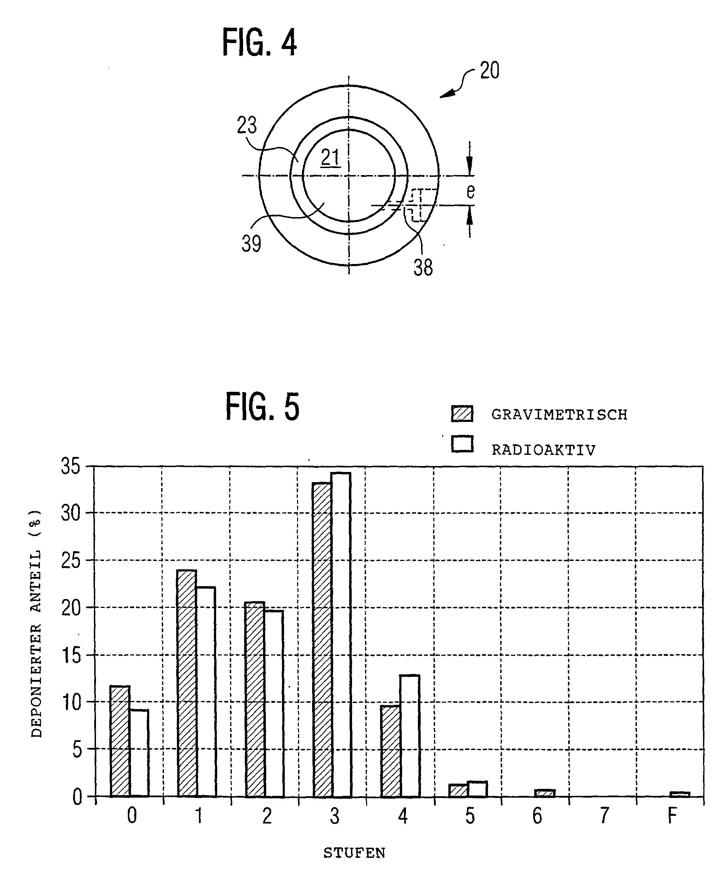

Mit 38 ist in den Fig. 2 bis 4 ein Einlaßkanal bezeichnet, der, wie in den Fig. 2 und 3 ersichtlich, im unteren Bereich des Innenraums 21 angeordnet und schräg nach unten auf die innere Bodenfläche 39 gerichtet ist. Aus der in Fig. 4 gezeigten Draufsicht auf den Grundkörper 20 ist weiterhin entnehmbar, daß die Einlaßöffnung 38 mit einer Exzentrizität e bezüglich der vertikalen Symmetrieachse X-X des Grundkörpers 20 angeordnet ist. Hierdurch läßt sich vorteilhaft ein zyklonartiges Verwirbeln der in dem Innenraum 21 befindlichen, nicht dargestellten pulverförmigen Trägerstoffe bei Einleiten der trockenen Aerosolteilchen erreichen.

In Fig. 5 ist eine graphische Darstellung einer mit einem Andersen-Impaktor gemessenen Partikelverteilung eines pulverförmigen Trägerstoffs in der Form dargestellt, daß ein Vergleich zwischen einer schwarz angegebenen gravimetrischen und einer weiß angegebenen radioaktiv gemessenen Verteilung gezeigt wird.

Zur Vornahme der Messungen wurden zunächst mit porösen Aerosolteilchen, die aus Albumin, DPPC und Estradiol bestanden, Natriumpertechnetat-Teilchen angelagert. Zwischen 5 und 50 mg der trockenen porösen Teilchen wurden in die Mischkammer gefüllt. Von vernebelten 10 MBq ^{99m}Tc gelangten zwischen 25% und 45% an die porösen Teilchen. Die Teilchengrößenverteilung einer repräsentativen Probe der Teilchen wurden vor und nach der Markierung mit einem 9-stufigen Impaktor gemessen, wobei die Teilchengrößenverteilung sowohl gravimetrisch (die Teilchen auf jeder Stufe des Impaktors wurden mittels Mikrowaage ausgewogen) und mittels Radioaktivitätsmessung untersucht wurden.

In Fig. 2 ist die Verteilung mit beiden Meßmethoden wiedergegeben. Man erkennt keinen signifikanten Unterschied der gravimetrisch bestimmten Verteilung und der radioaktiv bestimmten Verteilung. Hieraus ergibt sich, daß die Markierung die Pulverteilchen nicht beeinflußt und die Markierung die Teilchengrößenverteilung des Aerosols richtig wiederspiegelt.

## Patentansprüche

1. Verfahren zum trockenen Anbringen von Substanzen, insbesondere von Markierungssubstanzen und/oder aktiv wirksamen Substanzen, an inhalierbare pulverförmige Trägerstoffe, mit folgenden Verfahrensschritten:
a) Lösen der anzubringenden Substanz in einer Flüssigkeit;
b) Vernebeln des Fluids und anschließendes Trocknen der Aerosoltröpfchen zur Erzeugung von Aerosolteilchen; und
c) verwirbelndes Kontaktieren der Aerosolteilchen mit dem pulverförmigen Trägerstoff.

2. Verfahren nach Anspruch 1,
**dadurch gekennzeichnet,**
**daß** vor dem Schritt c) die Feuchte der Aerosolteilchen kontrolliert wird.

3. Verfahren nach Anspruch 2,
**dadurch gekennzeichnet,**
**daß** die Feuchte der Aerosolteilchen auf den jeweiligen Trägerstoff eingestellt wird.

4. Verfahren nach einem der vorangehenden Ansprüche,
**dadurch gekennzeichnet,**
**daß** das verwirbelnde Kontaktieren durch Ansaugen von zirkulär geführten Aerosolteilchen durch den Trägerstoff vorgenommen wird.

5. Verfahren nach Anspruch 4,
**dadurch gekennzeichnet,**
**daß** das Ansaugen der Aerosolteilchen durch den Trägerstoff über eine Rückhaltefilteranordnung durchgeführt wird.

6. Vorrichtung zum trockenen Anbringen von Substanzen, insbesondere von Markierungssubstanzen und/oder aktiv wirksamen Substanzen, an inhalierbare pulverförmige Trägerstoffe, mit
einem Aerosolgenerator (11), der einen Düsen-, Ultraschall- oder sonstigen Vernebler für die in einer Flüssigkeit gelöste anzubringende Substanz und eine Trocknungseinrichtung für die erzeugten Aerosolteilchen aufweist;
und mit einer Mischkammer (12) zur Aufnahme von pulverförmigem Trägerstoff und zu dessen verwirbelnder Kontaktierung mit zirkulär eingeleiteten trockenen Aerosolteilchen.

7. Vorrichtung nach Anspruch 6,
**dadurch gekennzeichnet,**
**daß** für das Einleiten trockener Aerosolteilchen und für die Verwirbelung in der Mischkammer (12) eine Vakuumpumpe (18) vorgesehen ist, die über eine Filteranordnung (17; 36, 37) mit der Mischkammer (12) verbunden ist.

8. Vorrichtung nach Anspruch 6 oder 7,
**dadurch gekennzeichnet,**
**daß** die Mischkammer (12) einen exzentrischen Einlaß (38) für die Aerosolteilchen aufweist.

9. Vorrichtung nach Anspruch 8,
**dadurch gekennzeichnet,**
**daß** der Einlaß (38) im unteren Bereich der Mischkammer (12) mit schräg nach unten geneigter Orientierung vorgesehen ist.

10. Vorrichtung nach einem der Ansprüche 6 bis 9,
**dadurch gekennzeichnet,**
**daß** zur Kontrolle der Feuchte der getrockneten Aerosolteilchen ein Feuchtesensor (14) vorgesehen ist.

11. Vorrichtung nach Anspruch 10,
**dadurch gekennzeichnet,**
**daß** der Feuchtesensor (14) in einer Verbindungsleitung (13) zwischen Aerosolgenerator (11) und Mischkammer (12) angeordnet ist.

12. Vorrichtung nach Anspruch 10 oder 11,
**dadurch gekennzeichnet,**
**daß** die Feuchte der getrockneten Aerosolteilchen zur Anpassung an die jeweilige Trägersubstanz mittels eines entsprechenden Ein- und Ausschaltens des Düsenverneblers im Aerosolgenerator (11) einstellbar ist.

13. Vorrichtung nach einem der Ansprüche 6 bis 12,
**dadurch gekennzeichnet,**
**daß** die Mischkammer (12) mittels eines Deckels (27) mit einem Stutzen (28) für den Anschluß einer Vakuumpumpe (18) abgedichtet verschließbar ist.

14. Vorrichtung nach Anspruch 13,
**dadurch gekennzeichnet,**
**daß** der Deckel (27) teilweise in einen oberen Abschnitt (22) der Mischkammer (12) einsetzbar ist.

15. Vorrichtung nach Anspruch 13 oder 14,
**dadurch gekennzeichnet,**
**daß** der Deckel (27) mittels einer Überwurfmutter (26) auf der Mischkammer (12, 20) befestigbar ist.

16. Vorrichtung nach einem der Ansprüche 13 bis 15,
**dadurch gekennzeichnet,**
**daß** zwischen dem Deckel (27) und der Mischkammer (12, 20) eine Filteranordnung (36, 37) angeordnet ist.

17. Vorrichtung nach einem der Ansprüche 7 bis 16,
**dadurch gekennzeichnet,**
**daß** die Filteranordnung einen Aufbau aus einem Feinfilter (37) mit einer Porengröße von ca. 1-3 µm und aus einem Stützfilter (36) hoher Festigkeit und einer Größe der Öffnungen zwischen ca. 50-500 µm aufweist.

18. Vorrichtung nach Anspruch 17,
**dadurch gekennzeichnet,**
**daß** der Feinfilter (37) aus einem Cellulosefilter besteht und der Stützfilter (36) mit einer Metallfolie gebildet ist.

19. Vorrichtung nach einem der Ansprüche 6 bis 18,
**dadurch gekennzeichnet,**
**daß** der Aerosolgenerator (11) zur Erzeugung einer Partikelgröße < 0,5 µm mit einer relativen Feuchte < 50% vorgesehen ist.

20. Vorrichtung nach einem der Ansprüche 8 bis 10.
**dadurch gekennzeichnet,**
**daß** als Feuchtesensor (19) ein Hygrometer vorgesehen ist.

## Claims

1. A method of dry application of substances, particularly marker substances and/or active substances, on inhalable pulverulent carrier substances, comprising the following steps:
(a) dissolving the substance to be applied in a liquid;
(b) vaporizing the fluid and subsequently drying the aerosol droplets for producing aerosol particles;
(c) establishing a whirling contact of the aerosol particles with said pulverulent carrier substance.

2. The method according to claim 1,
wherein prior to step (c) the moisture of said aerosol particles is checked.

3. The method according to claim 2,
wherein the moisture of said aerosol particles is adapted to the respective carrier substance.

4. The method according to a preceding claim,
wherein the establishment of said whirling contact is realized by aspiration of circulated aerosol particles through said carrier substance.

5. The method according to claim 4,
wherein the aspiration of said aerosol particles through said carrier substance is performed via a retaining filter array.

6. A device for dry application of substances, particularly marker substances and/or active substances, on inhalable pulverulent carrier substances, comprising
an aerosol generator (11) including a nozzle-type, ultrasonic or other vaporizer for the substance to be applied and dissolved in a liquid as well as a drying means for drying the aerosol particles so formed;
and a mixing chamber (12) for receiving pulverulent carrier substance and for contacting same by whirling with dry aerosol particles circulated and introduced therein.

7. Device according to claim 6,
wherein for the introduction of dry aerosol particles and for whirling in said mixing chamber (12) a vacuum pump (18) is provided which is connected to said mixing chamber (12) via a filter array (17; 36, 37).

8. Device according to claim 6 or 7,
wherein said mixing chamber (12) presents an eccentric inlet (38) for said aerosol particles.

9. Device according to claim 8,
wherein said inlet (38) is provided in the lower region of said mixing chamber (12) with an oblique downward orientation.

10. Device according to one of claims 6 - 9,
wherein for checking the moisture of said dried aerosol particles a moisture sensor (14) is provided.

11. Device according to claim 10,
wherein said moisture sensor (14) is arranged in a connecting line (13) between said aerosol generator (11) and said mixing chamber (12).

12. Device according to claim 10 or 11,
wherein the moisture of said dried aerosol particles can be adjusted for adaptation to the respective carrier substance by means of an appropriate on/of operation of said nozzle-type vaporizer in said aerosol generator (11).

13. Device according to one of claims 6 - 12,
wherein said mixing chamber (12) is adapted for being closed and sealed by means of a cover (27) having a connector (28) for connection of a vacuum pump (18).

14. Device according to claim 13,
wherein said cover (27) is adapted for being partly inserted into an upper section (22) of said mixing chamber (12).

15. Device according to claim 13 or 14,
wherein said cover (27) is adapted for being fastened by means of a swivel nut (26) on said mixing chamber (12, 20).

16. Device according to one of claims 13 to 15,
wherein a filter array (36, 37) is disposed between said cover (27) and said mixing chamber (12, 20).

17. Device according to one of claims 7 - 16,
wherein said filter array has a structure composed of a fine filter (37) having a mesh size of roughly 1 - 3 µm and a high-strength supporting filter (36) having a mesh size between roughly 50 and 500 mm.

18. Device according to claim 17,
wherein said fine filter (37) consists of a cellulose filter, and that said supporting filter (36) is formed with a metal sheet.

19. Device according to one of claims 6 - 18,
wherein said aerosol generator (11) is provided for producing a particle size of < 0.5 mm at a relative moisture of < 50%.

20. Device according to one of claims 8 - 10,
wherein a hygrometer is provided as moisture sensor (19).

## Revendications

1. Procédé pour l'application à sec de substances, notamment de substances de marquage et/ou de substances actives, sur des matières de support pulvérulentes pouvant être inhalées, présentant les étapes de procédé suivantes :
a) dissolution de la substance devant être appliquée, dans un liquide;
b) atomisation du fluide et séchage ultérieur des gouttelettes d'aérosol pour la production de particules d'aérosol; et
c) mise en contact, avec tourbillonnement, des particules d'aérosol avec la matière de support pulvérulente.

2. Procédé selon la revendication 1, **caractérisé en ce que** l'on contrôle l'humidité des particules d'aérosol avant l'étape (c).

3. Procédé selon la revendication 2, **caractérisé en ce qu'**on règle l'humidité des particules d'aérosol sur la matière de support respective.

4. Procédé selon l'une des revendications précédentes, **caractérisé en ce que** le contact avec tourbillonnement est réalisé par aspiration de particules d'aérosol, guidées selon un guidage circulaire, par la matière de support.

5. Procédé selon la revendication 4, **caractérisé en ce que** l'aspiration des particules d'aérosol est exécutée au moyen de la matière de support à travers un dispositif de filtre de retenue.

6. Dispositif pour appliquer à sec de substances, notamment de substances de marquage et/ou de substances actives, sur des matières de support pulvérulentes pouvant être inhalées, comportant
un générateur d'aérosol (11), qui comporte un atomiseur à buse, un atomiseur à ultrasons ou un autre type d'atomiseur pour la substance devant être appliquée, qui est dissoute dans un liquide, et un dispositif de séchage pour les particules d'aérosol produites; et
une chambre de mélange (12) servant à recevoir une matière de support sous forme pulvérulente et pour sa mise en contact, avec tourbillonnement, avec les particules d'aérosol sèches introduites en étant introduites circulairement.

7. Dispositif selon la revendication 6, **caractérisé en ce que** pour l'introduction de particules sèches d'aérosol et pour le tourbillonnement dans la chambre de mélange (12) il est prévu une pompe à vide (18), qui est reliée à la chambre de mélange (12) par l'intermédiaire d'un dispositif de filtre (17;36,37).

8. Dispositif selon la revendication 6 ou 7, **caractérisé en ce que** la chambre de mélange (12) comporte une entrée excentrique (38) pour les particules d'aérosol.

9. Dispositif selon la revendication 8, **caractérisé en ce que** l'entrée (38) est prévue dans la zone inférieure de la chambre de mélange (12) avec une orientation inclinée obliquement vers le bas.

10. Dispositif selon l'une des revendications 6 à 9, **caractérisé en ce que** pour le contrôle de l'humidité des particules d'aérosol sèches il est prévu un capteur d'humidité (14).

11. Dispositif selon la revendication 10, **caractérisé en ce que** le capteur d'humidité (14) est disposé dans une canalisation de liaison (13) entre le générateur d'aérosol (11) et la chambre de mélange (12).

12. Dispositif selon la revendication 10 ou 11, **caractérisé en ce que** l'humidité des particules d'aérosol sèches est réglable pour l'adaptation à la substance porteuse respective, à l'aide d'une activation et d'une désactivation correspondantes de l'atomiseur à buse dans le générateur d'aérosol (11).

13. Dispositif selon l'une des revendications 6 à 12, **caractérisé en ce que** la chambre de mélange (12) peut être fermée d'une manière étanche au moyen d'un couvercle (27) à l'aide d'une tubulure (28) pour le raccordement d'une pompe à vide (8).

14. Dispositif selon la revendication 13, **caractérisé en ce que** le couvercle (27) peut être inséré en partie dans une autre partie (22) de la chambre de mélange (12).

15. Dispositif selon l'une des revendications 13 ou 14, **caractérisé en ce que** le couvercle (27) peut être fixé à la chambre de mélange (12,20) par l'intermédiaire d'un écrou-chapeau (26).

16. Dispositif selon l'une des revendications 13 à 15, **caractérisé en ce qu'**un dispositif de filtre (36,37) est disposé entre le couvercle (27) et la chambre de mélange (12,20).

17. Dispositif selon l'une des revendications 7 à 16, **caractérisé en ce que** le dispositif de filtre possède un agencement constitué par un filtre fin (37) ayant des pores d'une taille d'environ 1 à 3 µm et par un filtre de support (36) présentant une grande solidité et possédant des ouvertures ayant une taille comprise entre environ 50 et 500 µm.

18. Dispositif selon la revendication 17, **caractérisé en ce que** le filtre fin (37) est formé par un filtre cellulosique et que le filtre auxiliaire (36) est formé par une feuille métallique.

19. Dispositif selon l'une des revendications 6 à 18, **caractérisé en ce que** le générateur d'aérosol (11) est destiné à produire des particules d'une taille < 0,5 µm avec une humidité relative < 50 %.

20. Dispositif selon l'une des revendications 8 à 10, **caractérisé en ce qu'**il est prévu un hygromètre en tant que capteur d'humidité (19).
